# EUROPÄISCHE PATENTSCHRIFT

(11) **EP 1 886 653 B1**
(45) Veröffentlichungstag und Bekanntmachung des Hinweises auf die Patenterteilung: **20.04.2011**
(21) Anmeldenummer: 06118554.2
(22) Anmeldetag: 07.08.2006
(51) Int. Cl.: A61F 9/007, A61M 3/02, A61B 17/34, A61M 1/00, F16L 37/04

(54) **Inzisionsvorrichtung für die Opthalmologie**
Incision device for ophthalmology
Dispositif d'incision destiné à l'ophtalmologie

(43) Veröffentlichungstag der Anmeldung: 13.02.2008
(73) Patentinhaber: Oertli-Instrumente AG, 9442 Berneck (CH)
(72) Erfinder: Di Nardo, Silvio, 9011 St. Gallen (CH); Nyffenegger, Bruno, 9436 Balgach (CH)
(74) Vertreter: Liebetanz, Michael

(56) Entgegenhaltungen:
- WO-A2-01/68016
- DE-U1- 29 710 025
- US-A- 3 659 607

## Beschreibung

### Technisches Gebiet der Erfindung

Die vorliegende Erfindung betrifft eine Inzisionsvorrichtung für die Ophthalmologie gemäss den Merkmalen des Oberbegriffs des Anspruchs 1.

### Stand der Technik

Solche Vorrichtungen sind aus dem Stand der Technik bekannt und werden durch Fachleute in einer Vielzahl von Eingriffen am Auge eingesetzt.

Unter dem Produktname "Pars Plana Microincision System PMS" vertreibt die Anmelderin eine solche Vorrichtung. Die Vorrichtung umfasst eine Vielzahl von chirurgischen Instrumenten. Typischerweise wird eine solche Vorrichtung für chirurgische Eingriffe in einem Auge eingesetzt. Dabei wird ein erster Trokar, auch als Führungsrohr bezeichnet, derart in das Auge eingesetzt, dass die Spitze des Trokars bis in den Glaskörperraum (Corpus vitreum) des Auges ragt, während das gegenüberliegende Ende als Schlauchanschluss bereit steht. An diesem ist ein Schlauch anschliessbar, über den und den ersten Trokar das Innere des Auges mit einer Infusion versorgbar ist, weshalb der Trokar auch als Infusionstrokar bezeichnet werden kann. Zudem werden meist zwei weitere Trokare eingesetzt. Über diese weiteren Trokare können dann entsprechende Instrumente oder Beleuchtungsvorrichtungen dem Auge zugeführt werden. Diese weiteren Trokare werden auch als Instrumententrokare bzw. Beleuchtungstrokare bezeichnet.

Diese Vorrichtung eignet sich sehr gut für Standardeingriffe, bei welchen keine grösseren Komplikationen zu erwarten sind. Es lassen sich aber keine Instrumente über den Infusionstrokar einführen. Wird an der Stelle, an welcher der Infusiontrokar eingesetzt ist, ein Instrumententrokar benötigt, so muss der Infusionstrokar entfernt und an einer neuen Stelle neu gesetzt werden. Dies erfordert auch eine neue Einsetzung des Instrumententrokars.

Ein weitere Vorrichtung ist aus WO 01/68016 bekannt. Hierbei wird eine Ausrichtungsvorrichtung mittels einem Setzinstrument in ein Auge eingesetzt. Die Ausrichtvorrichtung ist dabei zylinderförmig ausgebildet und umfasst im wesentlichen einen Flansch mit einem Nocken. Über die Ausrichtvorrichtung können dann weitere Instrumente, wie beispielsweise ein Schneidinstrument, in das Auge eingesetzt werden. Das Setzinstrument ist über den Nocken an der Ausrichtvorrichtung mit letzterer verbunden. Diese Verbindung lässt sich nach erfolgtem Einsetzen der Ausrichtvorrichtung trennen und das Setzwerkzeug kann entfernt werden. Die Ausrichtvorrichtung kann dann die weiteren Instrumente aufnehmen. Nachteilig ist, dass beim Entfernen des Setzinstrumentes durch Betätigung des Hebels zur Ausklinkung aus dem Nocken Querkräfte entstehen können, so dass die Öffnung im Auge vergrössert wird. Dies beeinträchtigt beispielsweise den späteren Heilungsprozess.

Die DE 297 10 025 offenbart eine Schnellkupplung für ein in ein Rohr einsetzbares Instrument. Die Schnellkupplung umfasst Betätigungselemente, welche in komplizierter Art und Weise mittels mechanischen Elementen mit einer Hülse verbunden sind. Aufgrund der Vielzahl von mechanischen Mittel ist die Miniaturisierung einer solchen Vorrichtung begrenzt und kann daher nicht in der Augenchirurgie eingesetzt werden.

### Darstellung der Erfindung

Ausgehend von diesem Stand der Technik liegt der Erfindung die Aufgabe zugrunde, eine Vorrichtung zu schaffen, welche eine verbesserte Kompatibilität zwischen Führungsrohr und in das Führungsrohr einzuführende Instrumente, wie Infusionsschläuche oder Beleuchtungsvorrichtungen, hat.

Eine weiteres Ziel der vorliegenden Erfindung ist, eine genannte Vorrichtung derart auszugestalten, dass diese einem Chirurgen grösstmöglichste Flexibilität in der Anwendung ermöglicht, so dass chirurgische Eingriffe an einem Auge mit einer grossen Effizienz erfolgen können.

Diese Aufgabe löst eine Vorrichtung mit den Merkmalen des Patentanspruchs 1. Vorteilhafte Ausgestaltungen der Erfindung sind in den abhängigen Ansprüchen angegeben.

Demgemäss ist ein Führungsrohraufsatz mit einem in ein Auge eingeführten Führungsrohr kuppelbar verbindbar und weist eine Mittelachse auf. Der Führungsrohraufsatz umfasst eine Instrumentenführung und eine Klemmeinheit. Die Klemmeinheit umfasst mindestens zwei, zur Mittelachse symmetrisch angeordnete Rastelemente, die durch ihnen jeweils zugeordnete Drucklaschen über einen in die Instrumentenführung führenden Übergang betätigbar sind.

Ein solcher Führungsrohraufsatz kann in besonders effizienter Weise mit einem eingesetzten oder einzusetzenden Führungsrohr verbunden werden. Zudem ermöglicht die Vorrichtung ein einfaches Trennen bzw. Auswechseln des Führungsrohraufsatzes.

Bevorzugterweise sind die Drucklaschen und die Rastelemente rotationssymmetrisch um die Mittelachse und koaxial zueinander angeordnet.

Aufgrund einer koaxialen Anordnung ist der Führungsrohraufsatz einfach bedienbar und ein Auftreten von radialen Kräften während der Bedienung kann verhindert werden.

Vorzugsweise ist der Übergang zwischen Instrumentenführung und Klemmeinheit eine Scheibe.

Vorzugsweise verfügen die Rastelemente auf ihrer zur Hauptachse geneigten Seite zumindest teilweise über eine Rastkerbe.

Die Rastkerbe ist besonders vorteilhaft, denn mittels der Rastkerbe kann eine besonders sichere Verbindung zwischen Führungsrohr und Führungsrohraufsatz erfolgen.

Bevorzugterweise verfügen die Rastelemente auf ihrer von der Hauptachse abgewandten Seite zumindest teilweise über eine Greifmulde.

Die Greifmulde erlaubt dem Benutzer eines solchen Führungsrohraufsatzes ein sicheres Ergreifen derselben.

Vorzugsweise weist die Instrumentenführung die Gestalt eines einstückig integrierten Anschlussstückes auf. Das Anschlussstück ist mit einem Instrument aus der Gruppe von Infusionsschlauch, Beleuchtungseinheit, Schneidinstrument, diathermisches Instrument, Beleuchtungsinstrument oder Lichtwellenleiter verbindbar.

Vorzugsweise weist die Instrumentenführung die Gestalt eines Anschlusseinsatzes auf. Der Anschlusseinsatz ist mit einem Instrument aus der Gruppe von Infusionsschlauch, Beleuchtungseinheit, Schneidinstrument, diathermisches Instrument, Beleuchtungsinstrument oder Lichtwellenleiter verbindbar.

Ein Set umfasst vorzugsweise mindestens eine, vorzugsweise mindestens drei, Führungsrohraufsätze, insbesondere für ein Instrument aus der Gruppe von Infusionsschlauch, Beleuchtungseinheit, Schneidinstrument, diathermisches Instrument oder Lichtwellenleiter und mindestens ein, vorzugsweise drei identische Führungsrohre. Die Führungsrohre bestehen aus einer Kanüle und einem Flansch, welcher an seinem von der Kanüle abgewandten Ende über einen für die Rastelemente komplementären Wulst verfügt.

Zwischen Führungsrohr und Führungsrohraufsatz ist vorzugsweise mindestens eine Dichtstelle zur Herstellung einer fluiddichten Verbindung zwischen Führungsrohr und Führungsrohraufsatz angeordnet.

Ein Führungsrohraufsatz und/oder einem Set wird vorzugsweise zum Setzen einer Infusion oder zum Einbringen eines Lichtwellenleiters in das Augeninnere oder zum Einbringen eines chirurgischen Werkzeugs in das Augeninnere verwendet.

Weitere vorteilhafte Ausführungsformen sind in den Unteransprüchen gekennzeichnet.

### Kurze Beschreibung der Zeichnungen

Die Erfindung wird im folgenden anhand der Zeichnungen näher beschrieben. Es zeigen:
- Fig. 1: eine Schnittdarstellung eines Auges mit einer Vorrichtung nach einem ersten Ausführungsbeispiel gemäss der vorliegenden Erfindung;
- Fig. 2: eine Detailansicht der Figur 1;
- Fig. 3: eine perspektivische Ansicht eines Führungsrohraufsatzes gemäss der vorliegenden Erfindung von oben;
- Fig. 4: eine perspektivische Ansicht des Führungsrohraufsatzes nach Figur 3 von unten;
- Fig. 5: eine Schnittdarstellung des Führungsrohraufsatzes nach Figuren 3 und 4;
- Fig. 6: eine Schnittdarstellung eines Auges mit einer Vorrichtung nach einem zweiten Ausführungsbeispiel gemäss der vorliegenden Erfindung;
- Fig. 7: eine Detailansicht der Figur 6;
- Fig. 8: eine perspektivische Ansicht eines Führungsrohraufsatzes gemäss der vorliegenden Erfindung nach einem zweiten Ausführungsbeispiel von oben;
- Fig. 9: eine perspektivische Ansicht des Führungsrohraufsatzes nach Figur 9 von unten;
- Fig. 10: eine Schnittdarstellung des Führungsrohraufsatzes nach Figuren 8 und 9; und
- Fig. 11: einen Einsatz zum Einsetzen in den Führungsrohraufsatz.

### Beschreibung eines Ausführungsbeispiels

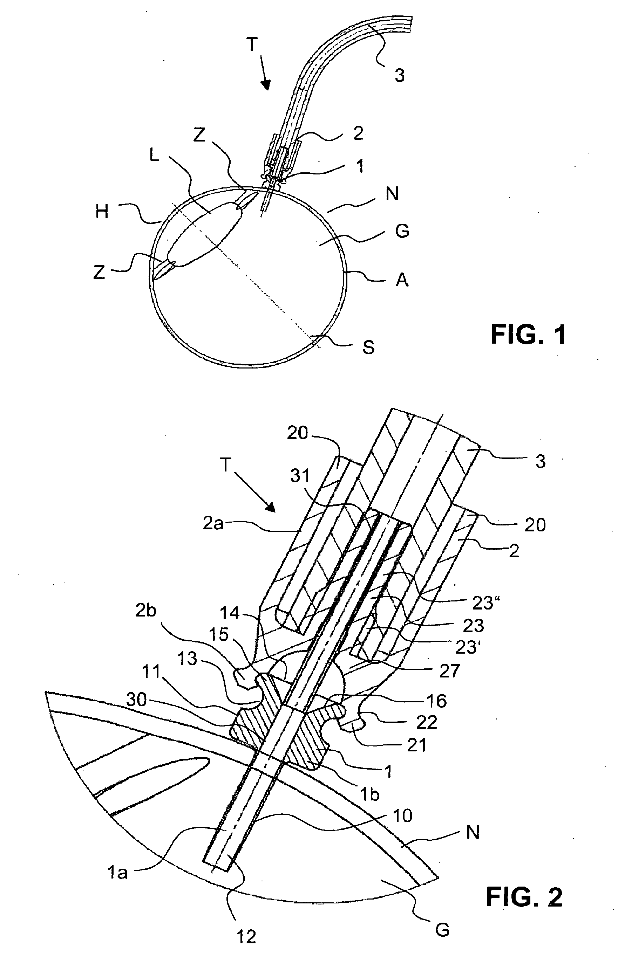

Figur 1 zeigt eine Schnittdarstellung eines menschlichen Auges mit einer in das Auge eingesetzten Vorrichtung T gemäss einem ersten Ausführungsbeispiel. Die einzelnen Elemente des Auges sind dabei in schematischer Weise dargestellt. Sichtbar sind der Glaskörper G, die Hornhaut H, die Linse L, die Netzhaut N, der Sehnerv S und die Zonulafasern Z. Im vorliegenden Ausführungsbeispiel ist die Vorrichtung T im Bereich, welcher an den Bereich der Zonulafasern Z angrenzend ist, eingeführt. Die Vorrichtung T kann aber auch an anderen Stellen in das Auge eingeführt werden. Die erfindungsgemässe Vorrichtung T umfasst im wesentlichen ein Führungsrohr 1, einen Führungsrohraufsatz 2 und beispielsweise ein Infusionsschlauch 3. Das Führungsrohr 1 kann beispielsweise auch als Trokar bezeichnet werden, wobei der Führungsrohraufsatz 2 dann als Trokarklammer bezeichnet wird. Der Infusionsschlauch 3 wird hier lediglich beispielhaft genannt und kann beispielsweise auch ein Lichtwellenleiter oder aber ein beliebiges Instrument sein.

Figur 2 zeigt eine detaillierte Schnittansicht der erfindungsgemässen Vorrichtung T.

Das Führungsrohr 1 ist im wesentlichen um eine Mittelachse 17 rotationssymmetrisch ausgestaltet und umfasst einen ersten Abschnitt 1a und einen zweiten Abschnitt 1b. Im ersten Abschnitt 1a hat das Führungsrohr 1 eine zylindrische Aussenform. Dieser erste Abschnitt 1a kann auch als Kanüle 10 bezeichnet werden. Der zweite Abschnitt 1b bildet sich durch einen an die Kanüle 10 angeformten Flansch 11. Der Flansch 11 weist einen grösseren Aussendurchmesser auf, als die Kanüle 10. Die Oberfläche des Flansches 11, welche dem Auge zugewandt ist, ist flach ausgestaltet.

Eine Öffnung 12 durchdringt das Führungsrohr 1 entlang der Längsachse oder Mittelachse 17 und bildet so einen Kanal durch den ersten Abschnitt 1a und den zweiten Abschnitt 1b. Im Bereich beim Austritt der Öffnung aus dem Flansch 11 weitet sich die Öffnung 12 über einen konischen Auslauf 16 auf. Die konische Ausgestaltung ist vorteilhaft, denn dies ermöglicht ein einfaches und sicheres Einführen von Instrumenten in die Öffnung 12 des Führungsrohrs 1.

Der Flansch 11 des zweiten Abschnittes 2b weist auf dessen nach aussen zugewandten Oberfläche eine mindestens teilweise oder ganz umlaufende Rille 13 auf. Durch die Anordnung der Rille 13 mit einem Abstand von der Oberfläche 14 des Flansches 11 wird eine Art umlaufender Wulst 15 gebildet. Der umlaufende Wulst 15 kann dabei als Rastmittel zur Verbindung des Führungsrohrs 1 mit dem Führungsrohraufsatz 2 verwendet werden.

Aufgrund der beschriebenen Ausgestaltung der umlaufenden Rille 13, welche auch als Pinzetten-Mulde bezeichnet werden kann, ist der Chirurg oder die ihm assistierenden Mitarbeiter in der Lage, das Führungsrohr 1 mit einer Pinzette gut zu ergreifen. Das Führungsrohr 1 kann auf einen nicht gezeigten Dorn aufgesetzt werden und mit einer über den Dorn aufbrachten Kraft über eine Öffnung in der Augenoberfläche in das Auge eingeführt werden. Die Öffnung ist vorzugsweise ein Schnitt, der in einem Winkel von 20° bis 70°, gemessen von der Senkrechten zur Augenoberfläche, ausgeführt wird. Besonders bevorzugt wird ein Winkel zwischen 30° und 60°. Der Schnitt wird vorzugsweise mittels einem chirurgischen Schneidinstrument, wie ein Skalpell, in die Augenoberfläche geschnitten. Aufgrund der winkligen Richtung des Schnittes wird bewirkt, dass die Öffnung in der Augenoberfläche möglichst klein bleibt bzw. eine Art Lappen gebildet wird, welcher dann beim Entfernen des Führungsrohrs 1 die Öffnung gut verschliesst. Daraus folgt ein für den Patienten vorteilhaften Wundverschluss. Dies führt im weiteren zu einem beschleunigten Heilungsprozess der Öffnung nach dem Eingriff. Das Führungsrohr 1 wird vorzugsweise in der Richtung des Schnittes oder in möglichst tangentialer Richtung eingeführt.

Der Dorn kann aber auch Teil eines nicht dargestellten Setzwerkzeuges zum Einsetzen des Führungsrohrs 1 sein. Zudem kann das Führungsrohr aber auch von Hand oder mit einer Pinzette eingesetzt werden.

Zudem ist es möglich, dass vorgängig ein Schneidinstrument, wie ein Messer, in die Öffnung 12 des Führungsrohres 1 eingeführt wird. Mit diesem Schneidinstrument kann dann die erforderliche Öffnung geschnitten werden, so dass das Führungsrohr anschliessend in das Auge eingeführt werden kann. Das heisst also, dass in einem einzigen Schritt die Öffnung im Gewebe gefertigt werden kann und dass gleichzeitig das Führungsröhrchen 1 eingesetzt werden kann.

Wie in Figur 2 erkennbar ist, ist das Führungsrohr 1 in der Darstellung mit der Kanüle 10 in das Auge eingesetzt. Das Führungsrohr 1 kann dabei soweit in das Auge eingeführt werden, bis der Flansch 11 auf der Oberfläche des Auges aufliegt. Der erste Abschnitt 1a weist eine Länge auf, die es der Kanüle erlaubt, bis in den Glaskörper G des Auges einzudringen.

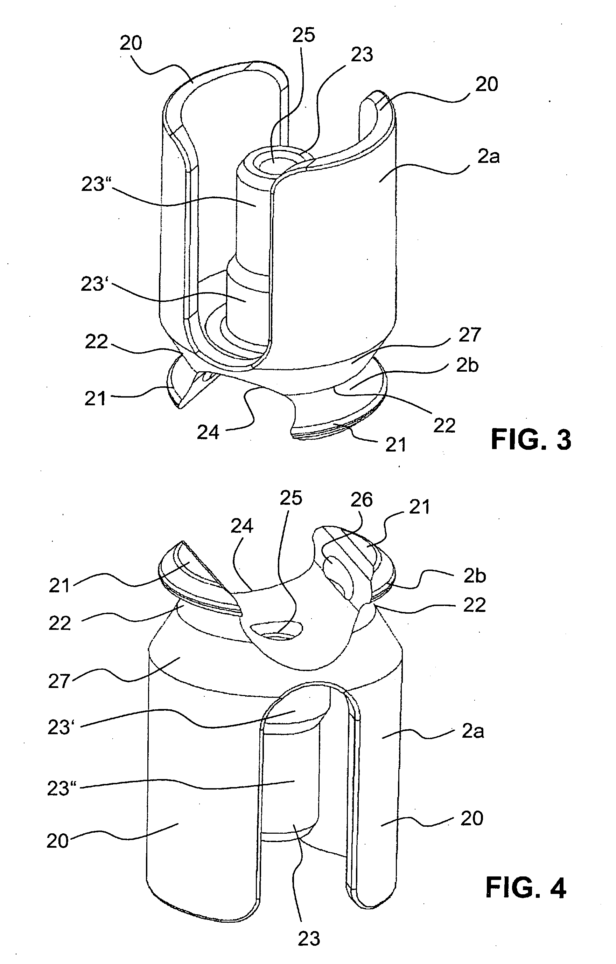

Der Führungsrohraufsatz 2 gemäss einem ersten Ausführungsbeispiel wird in Figuren 2 bis 5 in unterschiedlichen Ansichten dargestellt. Der Führungsrohraufsatz 2 umfasst einen Instrumentenführungsbereich oder ersten oberen Abschnitt 2a und einen Klemmbereich oder einen zweiten unteren Abschnitt 2b, wobei die beiden Abschnitte durch eine kreiszylindrische Scheibe 27 getrennt werden. Der Führungsrohraufsatz 2 ist vorzugsweise rotationssymmetrisch ausgestaltet.

Der obere Abschnitt 2a umfasst mehrere, hier zwei, äussere Drucklaschen 20 und einen als Zapfen ausgebildeten inneren Instrumentenanschluss oder Instrumentenführung 23. Der Zapfen 23 kann auch als einstückig integriertes Anschlussstück 23 bezeichnet werden. Sowohl der Zapfen 23 als auch die Drucklaschen 20 sind an der oberen Seite der kreiszylindrischen Scheibe 27 angeformt und erstrecken sich senkrecht zu dieser oberen Seite.

Der Zapfen 23 ist konzentrisch zur kreiszylindrischen Scheibe 27 angeordnet und hohlzylindrisch ausgestaltet. Das heisst der Zapfen weist eine Öffnung 25 auf. Die Mittelachse 17, welche auch die Mittelachse des Führungsrohraufsatzes 2 ist, verläuft durch das Zentrum der kreiszylindrischen Scheibe 27 und durch den Zapfen 23. In einem der kreiszylindrischen Scheibe 27 näher gelegenen ersten Bereich 23' weist der Zapfen 23 einen grösseren Aussendurchmesser als im oberen Bereich 23'' auf. Der Übergang zwischen vom oberen Bereich 23" zum unteren Bereich 23' ist konisch ausgestaltet.

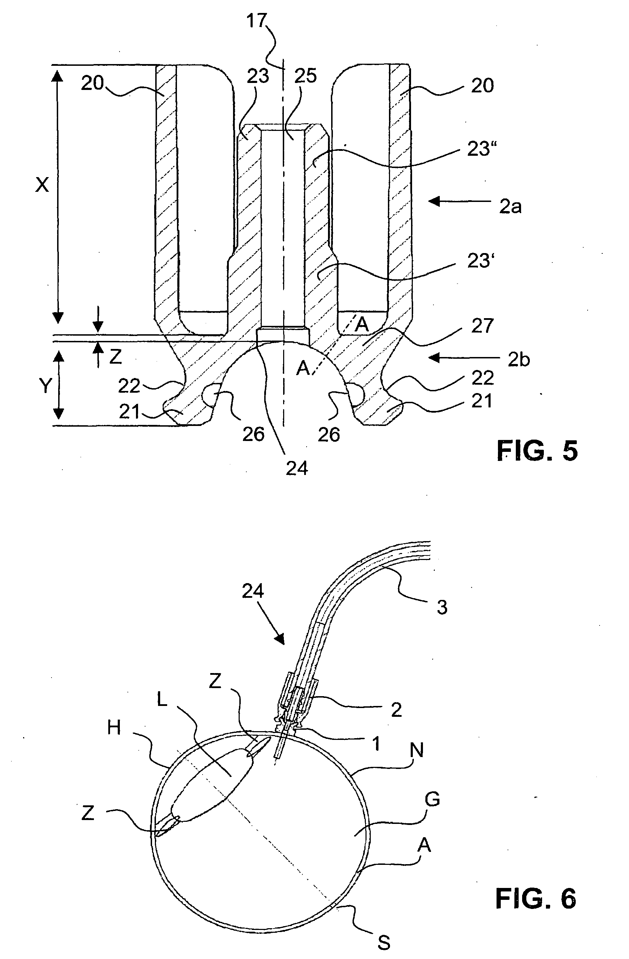

Die Drucklaschen 20 sind am äusseren Rand der Scheibe 27, vorzugsweise rotationssymmetrisch, angeformt und erstrecken sich im vorliegenden Ausführungsbeispiel über eine Bogenlänge, welche einen Winkel von 90° bis 150° begrenzt. Kleinere Winkel sind auch möglich. Das heisst also, dass im gezeigten Ausführungsbeispiel der Instrumentenanschluss 23 zwischen den beiden Drucklaschen 20 angeordnet ist. Wie in Figur 5 erkennbar ist, weisen die Drucklaschen 20 eine grössere Höhe als der Zapfen 23 auf.

Der untere Abschnitt 2b umfasst im wesentlichen mehrere, hier zwei, Rastelemente 21. Die Rastelemente 21 sind dabei an der unteren Seite der kreiszylindrischen Scheibe 27 angeformt. Die Rastelemente 21 sind dabei derart angeordnet, dass diese unterhalb und in gleicher Richtung zu den Drucklaschen 20 angeordnet sind. Vorzugsweise ist die Anzahl der Rastelemente 21 deshalb gleich der Anzahl der Drucklaschen 20. Ein Zwischenbereich 24 zwischen den Rastelementen 21 ist als Ausnehmung ausgestaltet. Die Ausnehmung 24 ist insbesondere in ihrer Dimension derart ausgestaltet, dass an der Stelle, welche in der Figur 5 mit einer Linie A-A gezeigt ist, eine kleine Querschnittsfläche entsteht. Wobei der Ausdruck kleine Querschnittsfläche dahingehend zu verstehen ist, dass der resultierende Querschnitt eine möglichst kleine Biegesteifigkeit aufweist und deshalb eine Verformung in diesem Bereich schon bei kleinen Kräften möglich ist. Die Verformung ist dabei als elastische Verformung zu verstehen. Um dieses Resultat zu erzielen, muss aber auch die kreiszylindrische Scheibe 27 eine möglichst geringe Dicke aufweisen. Aufgrund der geringen Dicke des für die Bewegung relevanten Querschnittes A-A der Scheibe 27 kann die Verbindung zwischen Scheibe 27 und Rastelementen 21 bzw. Drucklaschen 20 auch als Filmscharnier bezeichnet werden. Aufgrund der Ausgestaltung der Drucklasche 20 und dem Rastelement 21 bleibt die relative Verformung zwischen Drucklasche 20 und Rastelement 21 bei der Betätigung des Führungsrohraufsatzes 2 relativ gering oder gar nicht vorhanden. Mit anderen Worten heisst dies, dass die Drucklasche 20 und das Rastelement 21 bei der Betätigung starr zueinander bleiben. Vorzugsweise verformt sich demnach nur der Querschnitt A-A.

Die Öffnung 25 weist in ihrem unteren Bereich, das heisst in dem Bereich in welchem die Öffnung 25 in den Zwischenraum 24 mündet, einen etwas grösseren Durchmesser als im Bereich des Instrumentanschlusses 23 auf. Dieser grössere Durchmesser kann auch als Aufweitung bezeichnet werden. Dabei dient diese Aufweitung beispielsweise als Einführhilfe beim Einpressen oder Einfügen einer Kanüle. Zudem kann diese Aufweitung auch als Klebespalt verwendet werden, falls eine Kanüle mit dem Führungsrohraufsatz verklebt werden soll.

Die Rastelemente 21 weisen auf der in den Zwischenbereich 24 zugewandten Seite weiterhin Rastkerben 26 auf. Vorzugsweise haben die Rastkerben eine zum umlaufenden Wulst 15 des Führungsrohrs 1 komplementäre Oberfläche. Auf der Aussenseite weisen die Rastelemente 21 zudem Ausnehmungen oder Pinzettenmulden 22 auf. Diese Ausnehmungen oder Pinzettenmulden 22 ermöglichen ein Ergreifen des Führungsrohraufsatzes 2 mit einer Pinzette. Zudem haben sich die Ausnehmungen 22 für die Verformung der Rastelemente 21 aufgrund einer unten beschriebenen Einwirkung als vorteilhaft erwiesen haben.

Eine Kraft auf die beiden Drucklaschen 20, welche derart aufgebracht wird, dass sich die beiden Drucklaschen 20 aufeinander zu bewegen, bewirkt eine Aufspreizung der den Drucklaschen 20 gegenüberliegenden Rastelemente 21. Die Drucklaschen 20 lassen sich manuell mit zwei Fingern oder einer Pinzette oder einem anderen Werkzeug zusammendrücken. Beim Wegfall der Kraft, also im kraftlosen Zustand, gehen die beiden Drucklaschen 20 und demnach auch die Rastelemente 21 in ihre ursprüngliche Position zurück.

Die Drucklaschen 20 weisen in axialer Richtung eine grössere Länge als die Rastelemente 21 auf. Die Länge der Drucklaschen 20 ist dabei mit X und die Länge der Rastelemente 21 mit Y bezeichnet. Ein dazwischen liegendes Mass Z repräsentiert die ungefähre Dicke der Scheibe 27. Vorzugsweise ist das Verhältnis X:Y zwischen 10:1 und 1:1, besonders bevorzugt zwischen 6:1 und 2:1. Die beschriebenen Längenverhältnisse sind besonders vorteilhaft, denn aufgrund einer Hebelwirkung über die Scheibe 27 müssen die Drucklaschen 20 mit einem entsprechend grossen Weg zusammengedrückt werden, so dass sich die Rastelemente mit einem entsprechend kleineren weg nach aussen bewegen. Das heisst, ein unachtsames Lösen des Führungsrohraufsatzes 2 vom Führungsrohr 1 wird verhindert.

Im aufgespreizten Zustand kann dann der Führungsrohraufsatz 2 mit dem unteren Abschnitt 2b über das Führungsrohr 1 gestülpt werden. Dabei kommen die Rastkerben 26 des Führungsrohraufsatzes 2 auf den umlaufenden Wulst 15 des Führungsrohrs 1 zu liegen. Das heisst, dass im aufgespreizten Zustand der Innendurchmesser des Führungsrohraufsatzes 2 im unteren Bereich 2b grösser ist, als der Aussendurchmesser des Führungsrohrs 1. Somit wird verhindert, dass eine Axialkraft auf das Führungsrohr 1 wirkt, wenn der T Führungsrohraufsatz 2 mit dem Führungsrohr 1 verbunden wird. Aufgrund der symmetrischen Krafteinwirkung werden auch keine radialen Kräfte auf das Führungsrohr 1 aufgebracht. Sobald der Benutzer die Krafteinwirkung auf die Drucklaschen 20 beendet, kehren die Rastelemente 21, wie oben beschrieben, in ihre ursprüngliche Position zurück. Die Rastelemente 21 berühren dann den umlaufenden Wulst 15, so dass eine formschlüssige Verbindung entsteht. Der umlaufende Wulst wird dann von den Rastelementen 21 konzentrisch umgeben. Sind das Führungsrohr 1 und der Führungsrohraufsatz 2 in dieser Art und Weise miteinander im Eingriff, spricht man auch vom zusammengefügten Zustand.

Je nach Ausgestaltung der Dimensionen des umlaufenden Wulstes 15 bzw. der Rastelemente wird die formschlüssige Verbindung durch eine kraftschlüssige Verbindung unterstützt.

Der Aussendurchmesser des umlaufenden Wulstes 15 kann beispielsweise grösser gewählt werden, als der Innendurchmesser der Rastkerben 21 im kraftlosen Zustand. Dies bewirkt dann im zusammengefügten Zustand, dass aufgrund der mechanischen Ausgestaltung eine Klemmkraft vom Führungsrohraufsatz 2 auf das Führungsrohr 1 wirkt. Hier liegt also ein Kraftschluss und ein Formschluss vor. Ist die resultierende Reibkraft, welche aus der Klemmkraft und der Materialpaarung bzw. der Oberflächenbeschaffenheit des Führungsrohres 1 bzw. des Führungsrohraufsatzes resultiert, grösser als die zu überwindende Haftreibungskraft in radialer Richtung, so wird eine Rotationsbewegung des Führungsrohraufsatz 2 um das Führungsrohr 1 verhindert. Aufgrund der Dimensionierung des Durchmessers des umlaufenden Wulstes 15 und des Innendurchmessers der Rastelemente ist die Verbindung zwischen dem Führungsrohr 1 und dem Führungsrohraufsatz 2 sowohl kraftschlüssig als auch formschlüssig.

Falls der Durchmesser des umlaufenden Wulstes 15 kleiner gewählt wird, als der Innendurchmesser der Rastkerben 21 im kraftlosen Zustand, resultiert keine Restkraft. Somit wird in diesem Fall eine Rotationsbewegung des Führungsrohraufsatzes 2 um das Führungungsrohr 1 ermöglicht. Somit handelt es sich hier um eine formschlüssige Verbindung.

Aufgrund der rotationssymmetrischen Ausgestaltung kann der Führungsrohraufsatz 2 bezüglich des Führungsrohrs 1 eine in Bezug auf den Drehwinkel zwischen Führungsrohraufsatz 2 und Führungsrohr 1 beliebige Lage einnehmen. Dies ist besonders vorteilhaft, denn es erlaubt dem Chirurgen eine besonders effiziente und flexible Platzierung des Führungsrohraufsatzes 2. Derselbe Vorteil kann mit einem rotationssymmetrischen Führungsrohr und einem rechteckigen Führungsrohraufsatz erreicht werden, wenn an zwei gegenüberliegenden Seiten des Rechteckes zwei Drucklaschen 20 über eine in diesem Fall rechteckige Scheibe 27 mit Rastelementen 21 verbunden werden.

Durch ein erneutes Aufbringen einer Kraft auf die Drucklaschen 20 kommt es zu einer erneuten Aufspreizung der Rastelemente 21. Dabei kann der Führungsrohraufsatz 2 in einfacher Art und Weise vom Führungsrohr 1 entfernt werden. Auch hier treten keine axialen und radialen Kräfte auf das Führungsrohr 1 auf. Falls die gesamte Vorrichtung T aus dem Auge entfernt werden soll, kann dies auch ohne Entfernung des Führungsrohraufsatzes 2 vom Führungsrohr 1 erfolgen. Dabei kann eine auf den Führungsrohraufsatz 2 wirkende Zugkraft über die Verbindung zwischen Führungsrohraufsatz 2 und Führungsrohr 1 direkt auf das Führungsrohr 1 übertragen werden. Durch die Zugkraft wird dann das Führungsrohr 1 aus der Öffnung im Auge entfernt. Die Zugkraft kann entweder von Hand direkt an den Drucklaschen 20 oder aber über eine Pinzette über die Pinzettenmulden 22 erfolgen. Der Vorgang der Entfernung erfolgt dann bei einer Infusion ohne Flüssigkeitsverlust, da das Führungsrohr 1 und der Führungsrohraufsatz 2 hier eine Einheit bilden.

Der Vorgang des Verbindens und des Trennens des Führungsrohraufsatzes 2 mit einem Führungsrohr 1 ist, wie oben beschrieben, besonders vorteilhaft, denn es kommt zu keinen axialen Kräften (Zugkräfte oder Druckkräfte) bei den beiden Vorgängen. Der Chirurg kann also einen Führungsrohraufsatz 2 in einfacher Art und Weise mit einem Führungsrohr 1 verbinden und wieder trennen. Dies ermöglicht einen flexiblen Einsatz von einem Führungsrohraufsatz 2 an verschiedenen im Auge eingesetzten Führungsrohren 1.

Wie bereits erwähnt weist der Instrumentenanschluss eine Öffnung 25 auf, welche sich durch den Zapfen 23 bis hin zum Zwischenbereich 24 erstreckt. Durch diese Öffnung können Gegenstände oder Fluide vom ersten Abschnitt 2a in den zweiten Abschnitt 2b geführt werden.

Wie dies in Figur 2 gezeigt wird, kann beispielsweise ein Infusionsschlauch 3 über den Zapfen 23 gestülpt werden. Der Schlauch umfasst zudem eine Kanüle 31. Die Kanüle 31 weist dabei eine derart dimensionierte Länge auf, dass die Kanüle 31 bis in die Öffnung 12 des Führungsrohres 1 ragen kann. Als Infusionsschläuche eignen sich insbesondere PVC- oder Silikonschläuche. Ein Führungsrohraufsatz 2 für eine Infusion wird als Infusions-Führungsrohraufsatz bezeichnet.

Beispielsweise kann das radiale Spiel zwischen Aussendurchmesser der Kanüle 31 und des Innendurchmessers des Führungsrohrs 1 maximal 0.02 mm betragen. Dabei wird eine für diese Anwendung ausreichende Dichtheit erzielt. Ein Austreten von Flüssigkeit ist nur noch tropfenweise möglich.

Alternativ kann der Führungsrohraufsatz 2 auch mit einem Lichtwellenleiter zur Beleuchtung des Inneren des Auges verbunden sein. Ein Führungsrohraufsatz 2 zur Beleuchtung wird als Beleuchtungs-Führungsrohraufsatz bezeichnet. Ein Lichtwellenleiter ragt dann beispielsweise in den ersten Bereich 1a des Führungsrohres 1.

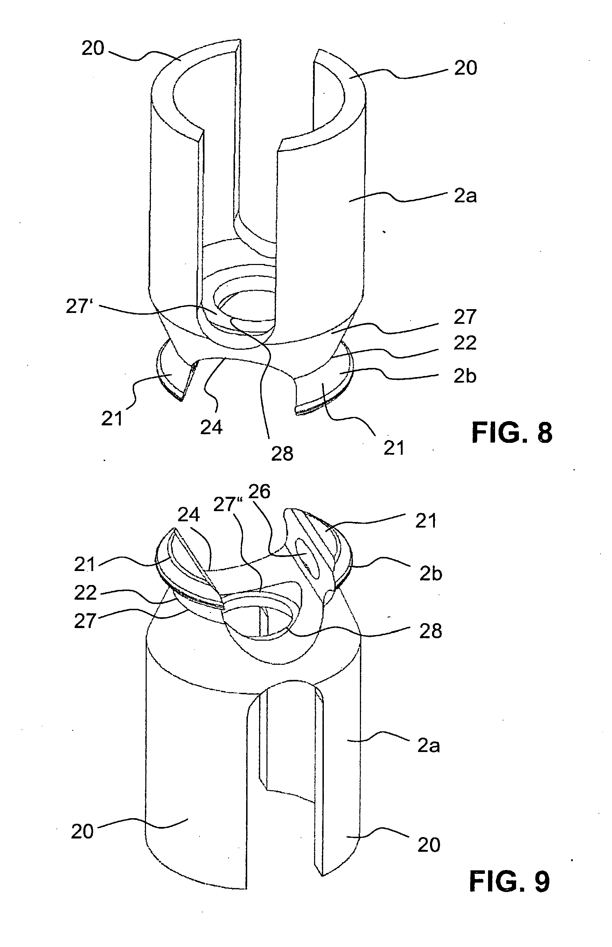

Figuren 6 bis 11 zeigen Darstellungen der erfindungsgemässen Vorrichtung mit dem Führungsrohr 1 und des Führungsrohraufsatzes 2 nach einem zweiten Ausführungsbeispiel gemäss der vorliegenden Erfindung. Dabei ist das Führungsrohr 1 zu dem Führungsrohr 1 vom ersten Ausführungsbeispiel identisch ausgestaltet. Zudem umfasst die Vorrichtung in diesem Ausführungsbeispiel ein Anschlussstück 4. Gleiche Teile sind mit identischen Bezugszeichen versehen.

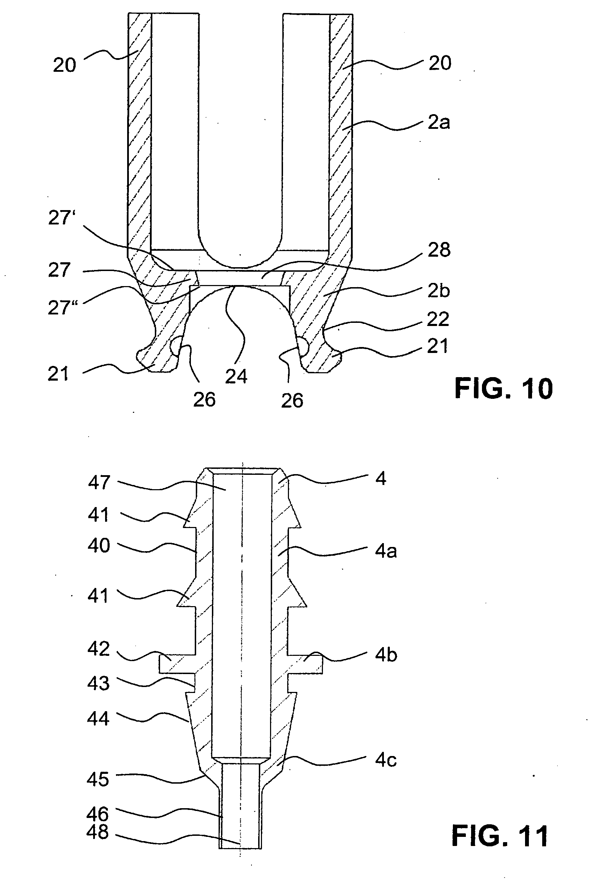

Figur 11 zeigt den Anschlusseinsatz 4. Der Anschlusseinsatz 4 ist im wesentlichen zylindrisch ausgestaltet und weist in axialer Richtung einen ersten Abschnitt 4a, einen zweiten Abschnitt 4b und einen dritten Abschnitt 4c auf.

Der erste Abschnitt 4a ist beispielsweise mit einem Infusionsschlauch oder einem Lichtwellenleiter verbindbar. An der im wesentlichen zylinderförmig ausgestalteten Aussenseite 40 weist der erste Abschnitt 4a eine Mehrzahl, hier zwei, Erhöhungen 41 auf. Die Aussenseite 40 kann auch als Instrumentenführung bezeichnet werden. Diese ringförmig umlaufenden oder unterbrochenen Erhöhungen 41 haben im Querschnitt die Form eines Keils und erhöhen aufgrund ihrer Form die Haftkraft zwischen Infusionsschlauch 3 und Anschlusseinsatz 4.

Der zweite Abschnitt 4b weist einen Flansch 42 auf. Der Flansch 42 hat dabei einen Durchmesser, welcher grösser ist als der Durchmesser des ersten Abschnittes. Über eine zylindrische Aussenform 43 mit einem kleineren Durchmesser als der Durchmesser des Flansches 42 geht der zweite Abschnitt 4b in den dritten Abschnitt 4c über.

Der dritte Abschnitt 4c bildet den Abschluss des Anschlusseinsatzes 4. Der dritte Abschnitt 4c umfasst im wesentlichen zwei Konen 44, 45 und einen zylindrischen Abschluss 46. Der erste Konus 44 erstreckt sich nach der zylindrischen Aussenform 43. Dabei weist dieser Konus 44 zu Beginn einen grösseren Durchmesser als die zylindrische Aussenform 43 auf. Der Durchmesser des Konus 44 verkleinert sich in konstanter Weise. Der Konus 45 ist am Konus 44 angeformt und verjüngt sich ebenfalls. Allerdings ist verjüngt sich der Konus 45 in steilerer Weise als der Konus 44. Ein zylinderförmiger Zapfen 46 bildet den Abschluss des dritten Abschnittes.

Im weiteren weist der Anschlusseinsatz 4 eine Öffnung 47 auf, welche sich entlang einer Mittelachse 48 durch alle drei Abschnitte 4a, 4b, 4c erstreckt.

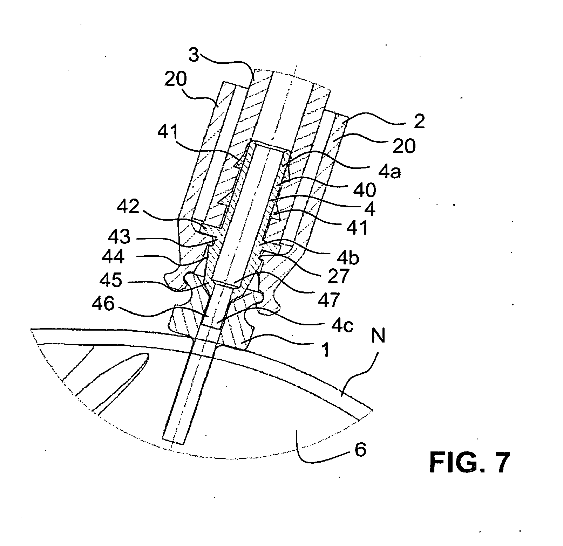

Figuren 7 bis 10 zeigen den Führungsrohraufsatz 2 nach dem zweiten Ausführungsbeispiel. Analog zum ersten Ausführungsbeispiel weist der Führungsrohraufsatz 2 ebenfalls einen oberen Abschnitt 2a und einen unteren Abschnitt 2b auf. Der obere Abschnitt 2a und der untere Abschnitt 2b können auch als Klemmeinheit bezeichnet werden. Der obere Abschnitt 2a weist, wie schon im ersten Ausführungsbeispiel mehrere, hier zwei, Drucklaschen 20 auf. Eine Öffnung 28 durchdringt die kreiszylindrische Scheibe 27. Die Öffnung kann ebenfalls als Instrumentenführung bezeichnet werden. Die Öffnung 28 weist dabei eine sich vom oberen Abschnitt 2a zum unteren Abschnitt 2 verjüngende Form auf. Der Anschlusseinsatz 4 kann in diese Öffnung 28 eingeführt werden. Die verjüngende Form und die konische Ausgestaltung des Anschlusseinsatzes 4 im dritten Anschnitt 4c unterstützt den Vorgang des Einführens. Dabei kommt die eine Seite des Flansches 42 auf der oberen Seite 27' der kreiszylindrischen Scheibe 27 und die zylindrische Aussenform 43 kommt auf die Oberfläche der Öffnung 28 zu liegen. Der Übergang von der zylindrischen Aussenform 43 zum Konus 44 liegt im eingeführten Zustand auf der unteren Seite 27" der kreiszylindrischen Scheibe 27 auf. Der Anschlusseinsatz 4 ist somit gegen axiale Bewegungen gesichert, kann aber bei grossen axialen Kräften, wie diese beispielsweise bei einer gewünschten Trennung von Anschlusseinsatz 4 und Führungsrohraufsatz 2 auftreten, dennoch vom Führungsrohraufsatz 2 getrennt werden. Der oben beschriebene Effekt des Filmscharniers ist hier immer noch vorhanden. Die kreiszylindrische Scheibe 27 kann hier als das Filmscharnier wirken. Die kreiszylindrische Scheibe kann auch als Torus oder als einem Torus ähnlich beschrieben werden.

Figur 7 zeigt den mit dem Führungsrohr 1 verbundene Führungsrohraufsatz 2 mit dem Anschlusseinsatz 4. Hier ist auch zu erkennen, dass der Anschlusseinsatz gemäss dem zweiten Ausführungsbeispiel derart ausgestaltet ist, dass der Konus 45 auf den konischen Auslauf 16 des Führungsrohrs 1 zu liegen kommt. Der zylindrische Zapfen 46 ragt in die Öffnung 12 des Führungsrohrs 1 hinein. Durch das Aufliegen des Konus 45 auf dem Auslauf 16 und das Hineinragen des hohlzylindrischen Zapfens 46 in die Öffnung 12 wird eine fluiddichte Verbindung zwischen Anschlusseinsatz 4 und Führungsrohr 1 bereitgestellt.

Bei einer Operation an einem Auge setzt der Chirurg typischerweise mehrere, insbesondere drei, Führungsrohre 1 gemäss der vorliegenden Erfindung. Dabei übernehmen die Führungsrohre 1 unterschiedlichste Funktionen. Die Art der Verbindung zwischen Führungsrohr 1 und Führungsrohraufsatz 2 erlaubt dem Chirurgen oder seiner Assistenz ein einfaches und effizientes Wechseln des Führungsrohraufsatzes 2. Falls also anstelle des Führungsrohraufsatzes mit der Infusion ein Führungsrohraufsatz 2 mit der Beleuchtungsvorrichtung verwendet werden soll, kann der Führungsrohraufsatz mit der Infusion in einfacher Art und Weise mit dem Führungsrohraufsatz mit der Beleuchtungsvorrichtung ausgetauscht werden.

Vorzugsweise werden die Führungsrohraufsätze 2 gemäss der vorliegenden Erfindung aus Kunststoff, insbesondere Polykarbonat oder Polyethylen, gefertigt. Vorzugsweise wird der Führungsrohraufsatz 2 mittels einem Spritzgussverfahren hergestellt. Besonders bevorzugt ist der eingesetzte Kunststoff transparent ausgestaltet und ermöglicht somit, dass der Chirurg einen besseren Überblick über die Einstichstelle hat.

In weiteren Ausführungsformen kann der Führungsrohraufsatz 2 auch aus einem anderen Material, wie beispielsweise Metall gefertigt sein. Insbesondere kommen hier beispielsweise rostfreier Stahl, Titan oder Titanlegierungen zur Anwendung. Denkbar sind auch andere bioverträgliche Metalle und Materialien.

Die Führungsrohre werden vorzugsweise aus einem metallischen Werkstoff, wie beispielsweise rostfreier Stahl, Titan oder Titanlegierungen. Denkbar sind auch andere bioverträgliche Metalle und Materialien. Ebenfalls können diverse Kunststoffe, wie Polykarbonat oder Polyethylen, eingesetzt werden.

In weiteren nicht gezeigten Ausführungsbeispielen lassen sich auch anderen Instrumenten, wie beispielsweise Schneidwerkzeuge, oder weitere Flüssigkeitstransfervorrichtungen am Führungsrohr 1 befestigen. Dabei können die Schneidwerkzeug mit dem Führungsrohraufsatz 2 verbunden werden oder aber in dieser integriert sein.

In einem weiteren nicht gezeigten Ausführungsbeispiel ist der Führungsrohraufsatz 2 in deren Zwischenraum 24 mit einem an der unteren Seite der zylindrischen Scheibe angeformten Zapfen ausgestaltet. Mit einem derartigen Führungsrohraufsatz lässt sich eine Öffnung 12 in einem Führungsrohr fluiddicht verschliessen. Um die Dichtheit zu erhöhen kann der Zapfen derart ausgebildet sein, dass Teile des Zapfens auf den konischen Auslauf 16 zu liegen kommen. Zusätzlich können elastische Elemente, wie beispielsweise O-Ringe vorgesehen sein, welche die Dichtwirkung zusätzlich erhöhen.

### Bezugszeichenliste

- A: Lederhaut
- G: Glaskörper
- H: Hornhaut
- L: Linse
- N: Netzhaut
- S: Sehnerv
- Z: Zonulafasern

- 1: Führungsrohr
- 2: Führungsrohraufsatz
- 3: Infusionsschlauch
- 4: Anschlusseinsatz

- 10: Kanüle
- 11: Flansch
- 12: Öffnung
- 13: umlaufende Rille
- 14: Oberseite Flansch
- 15: umlaufender Wulst
- 16: konischer Auslauf
- 17: Mittelachse

- 20: Drucklaschen
- 21: Rastelemente
- 22: Pinzettenmulde/Ausnehmung
- 23: Instrumentenanschluss
- 24: Zwischenraum
- 25: Öffnung
- 26: Rastkerben
- 27: kreiszylindrische Scheibe
- 28: Öffnung

- 30: Dichtstelle
- 31: Kanüle

- 40: Aussenseite
- 41: Erhöhungen
- 42: Flansch
- 43: zylindrische Aussenform
- 44: Konus
- 45: Konus
- 46: Zapfen
- 47: Öffnung
- 48: Mittelachse

## Patentansprüche

1. Führungsrohraufsatz (2), welcher mit einem in ein Auge eingeführtes Führungsrohr (1) kuppelbar verbindbar ist und eine Mittelachse aufweist, **dadurch gekennzeichnet, dass** der Führungsrohraufsatz (2) eine Instrumentenführung (23; 28, 40) und eine Klemmeinheit (2a, 2b) umfasst, wobei die Klemmeinheit (2a, 2b) mindestens zwei, zur Mittelachse symmetrisch angeordnete Rastelemente (21) umfasst, die durch ihnen jeweils zugeordnete Drucklaschen (20) über einen in die Instrumentenführung (23; 28, 40) führenden Übergang (27) betätigbar sind.

2. Führungsrohraufsatz (2) nach Anspruch 1, **dadurch gekennzeichnet, dass** der Führungsrohraufsatz (2) einstückig ausgebildet ist.

3. Führungsrohraufsatz (2) nach Anspruch 1 oder Anspruch 2, **dadurch gekennzeichnet, dass** die Drucklaschen (20) und die Rast-elemente (21) rotationssymmetrisch um die Mittelachse und koaxial zueinander angeordnet sind.

4. Führungsrohraufsatz (2) nach einem der vorhergehenden Ansprüche, **dadurch gekennzeichnet, dass** der Übergang zwischen Instrumentenführung (23; 28, 40) und Klemmeinheit (2a, 2b) eine Scheibe (27) ist.

5. Führungsrohraufsatz (2) nach einem der vorhergehenden Ansprüche, **dadurch gekennzeichnet, dass** die Rastelemente (21) auf ihrer zur Hauptachse geneigten Seite zumindest teilweise über eine Rastkerbe (26) verfügen.

6. Führungsrohraufsatz (2) nach einem der vorhergehenden Ansprüche, **dadurch gekennzeichnet, dass** die Rastelemente (21) auf ihrer von der Hauptachse abgewandten Seite zumindest teilweise über eine Greifmulde (22) verfügen.

7. Führungsrohraufsatz (2) nach einem der vorhergehenden Ansprüche, **dadurch gekennzeichnet, dass** die Instrumentenführung (23; 28, 40) die Gestalt eines einstückig integrierten Anschlussstückes (23) aufweist, welches Anschlussstück (23) mit einem Instrument aus der Gruppe von Infusionsschlauch, Beleuchtungseinheit, Schneidinstrument, diathermisches Instrument, Beleuchtungsinstrument oder Lichtwellenleiter verbindbar ist.

8. Führungsrohraufsatz (2) nach einem der vorhergehenden Ansprüche, **dadurch gekennzeichnet, dass** die Instrumentenführung (23; 28, 40) die Gestalt eines Anschlusseinsatzes (4) aufweist, welcher Einsatz (4) mit einem Instrument aus der Gruppe von Infusionsschlauch, Beleuchtungseinheit, Schneidinstrument, diathermisches Instrument, Beleuchtungsinstrument oder Lichtwellenleiter verbindbar ist

9. Set mit mindestens einem, vorzugsweise mindestens drei, Führungsrohraufsätzen (2), insbesondere für ein Instrument aus der Gruppe von Infusionsschlauch, Beleuchtungseinheit, Schneidinstrument, diathermisches Instrument oder Lichtwellenleiter, nach einem der vorstehenden Ansprüche und mindestens einem, vorzugsweise drei, identischen Führungsrohren (1), wobei die Führungsrohre (1) aus einer Kanüle (10) und einem Flansch (11) bestehen, welcher an seinem von der Kanüle (10) abgewandten Ende über einen für die Rastelemente (21) komplementären Wulst verfügt.

10. Set nach Anspruch 9, **dadurch gekennzeichnet, dass** zwischen Führungsrohr (1) und Führungsrohraufsatz (2) mindestens eine Dichtstelle (30, 16) zur Herstellung einer fluiddichten Verbindung zwischen Führungsrohr (1) und Führungsrohraufsatz (2) angeordnet ist.

## Claims

1. Pilot tube attachment (2), which can be connected by coupling to a pilot tube (1) inserted into an eye and has a centre axis, **characterized in that** the pilot tube attachment (2) comprises an instrument guide (23; 28, 40) and a clamp unit (2a, 2b), the clamp unit (2a, 2b) comprising at least two catch elements (21) which are arranged symmetrically with respect to the centre axis and which can be actuated by respectively associated pressure tabs (20) via a transition (27) leading into the instrument guide (23; 28, 40).

2. Pilot tube attachment (2) according to claim 1, **characterized in that** the pilot tube attachment (2) is provided as a single piece.

3. Pilot tube attachment (2) according to Claim 1 or 2, **characterized in that** the pressure tabs (20) and the catch elements (21) are arranged rotationally symmetrically about the centre axis and are coaxial with respect to one another.

4. Pilot tube attachment (2) according to one of the preceding claims, **characterized in that** the transition between instrument guide (23; 28, 40) and clamp unit (2a, 2b) is a disc (27).

5. Pilot tube attachment (2) according to one of the preceding claims, **characterized in that** the catch elements (21), on their side inclined towards the main axis, at least partially have a locking notch (26).

6. Pilot tube attachment (2) according to one of the preceding claims, **characterized in that** the catch elements (21), on their side directed away from the main axis, at least partially have a gripping groove (22).

7. Pilot tube attachment (2) according to one of the preceding claims, **characterized in that** the instrument guide (23; 28, 40) has the form of an integrated connector piece (23), which connector piece (23) can be connected to an instrument from the group of infusion line, illuminating unit, cutting instrument, diathermy instrument, illuminating instrument or optical waveguide.

8. Pilot tube attachment (2) according to one of the preceding claims, **characterized in that** the instrument guide (23; 28, 40) has the form of a connector insert (4), which insert (4) can be connected to an instrument from the group of infusion line, illuminating unit, cutting instrument, diathermy instrument, illuminating instrument or optical waveguide.

9. Set with at least one pilot tube attachment (2), preferably with at least three pilot tube attachments (2), in particular for an instrument from the group of infusion line, illuminating unit, cutting instrument, diathermy instrument or optical waveguide, according to one of the preceding claims, and with at least one pilot tube (1), preferably with three identical pilot tubes (1), said pilot tubes (1) consisting of a cannula (10) and a flange (11) which, at its end directed away from the cannula (10), has a complementary bead for the catch elements (21).

10. Set according to Claim 9, **characterized in that**, between pilot tube (1) and pilot tube attachment (2), there is at least one seal (30, 16) for producing a fluid-tight connection between pilot tube (1) and pilot tube attachment (2).

## Revendications

1. Elément tubulaire de guidage (2), qui peut être relié par couplage à un tube de guidage (1) introduit dans un oeil et qui présente un axe central, **caractérisé en ce que** l'élément tubulaire de guidage (2) comprend un guide d'instrument (23; 28, 40) et une unité de serrage (2a, 2b), dans lequel l'unité de serrage (2a, 2b) comprend au moins deux éléments d'encliquetage (21) disposés de façon symétrique par rapport à l'axe central, qui peuvent être actionnés par des pattes de pression (20) qui leur sont respectivement associées, par l'intermédiaire d'une nervure (27) menant au guide d'instrument (23; 28, 40).

2. Elément tubulaire de guidage (2) selon la revendication 1, **caractérisé en ce que** l'élément tubulaire de guidage (2) est réalisé d'un seul tenant.

3. Elément tubulaire de guidage (2) selon la revendication 1 ou 2, **caractérisé en ce que** les pattes de pression (20) et les éléments d'encliquetage (21) sont disposés de façon symétrique en rotation autour de l'axe central et de façon coaxiale les uns par rapport aux autres.

4. Elément tubulaire de guidage (2) selon l'une quelconque des revendications précédentes, **caractérisé en ce que** la nervure entre le guide d'instrument (23; 28, 40) et l'unité de serrage (2a, 2b) est un disque (27).

5. Elément tubulaire de guidage (2) selon l'une quelconque des revendications précédentes, **caractérisé en ce que** les éléments d'encliquetage (21) sont au moins partiellement munis d'une gorge d'encliquetage (26) sur leur côté incliné vers l'axe principal.

6. Elément tubulaire de guidage (2) selon l'une quelconque des revendications précédentes, **caractérisé en ce que** les éléments d'encliquetage (21) sont au moins partiellement munis d'une moulure de prise (22) sur leur côté situé à l'opposé de l'axe principal.

7. Elément tubulaire de guidage (2) selon l'une quelconque des revendications précédentes, **caractérisé en ce que** le guide d'instrument (23; 28, 40) présente la forme d'une pièce de raccordement (23) intégrée d'un seul tenant, laquelle pièce de raccordement (23) peut être reliée à un instrument du groupe comprenant un tuyau de perfusion, une unité d'éclairage, un instrument d'incision, un instrument diathermique, un instrument d'éclairage ou un guide d'ondes lumineuses.

8. Elément tubulaire de guidage (2) selon l'une quelconque des revendications précédentes, **caractérisé en ce que** le guide d'instrument (23; 28, 40) présente la forme d'un insert de raccordement (4), lequel insert (4) peut être relié à un instrument du groupe comprenant un tuyau de perfusion, une unité d'éclairage, un instrument d'incision, un instrument diathermique, un instrument d'éclairage ou un guide d'ondes lumineuses.

9. Ensemble comprenant au moins un, de préférence au moins trois éléments tubulaires de guidage (2), en particulier pour un instrument du groupe comprenant un tuyau de perfusion, une unité d'éclairage, un instrument d'incision, un instrument diathermique ou un guide d'ondes lumineuses selon l'une quelconque des revendications précédentes, et au moins un, de préférence trois tubes de guidage identiques (1), dans lequel les tubes de guidage (1) se composent d'une canule (10) et d'une bride (11), qui est munie d'un bourrelet complémentaire aux éléments d'encliquetage (21) sur son extrémité située à l'opposé de la canule (10).

10. Ensemble selon la revendication 9, **caractérisé en ce qu'**au moins une zone d'étanchéité (30, 16) est disposée entre le tube de guidage (1) et l'élément tubulaire de guidage (2) pour former un assemblage étanche au fluide entre le tube de guidage (1) et l'élément tubulaire de guidage (2).
